# FASCICULE DE BREVET EUROPEEN

(11) **EP 4 003 137 B1**
(45) Date de publication et mention de la délivrance du brevet: **28.06.2023**
(21) Numéro de dépôt: 20820476.8
(22) Date de dépôt: 31.07.2020
(51) Int. Cl.: A61B 1/00, A61B 1/273, A61B 1/018, A61B 1/015

(54) **SURTUBE ET DISPOSITIF MÉDICAL UTILISANT CE SURTUBE**
ÜBERTUBUS UND MEDIZINPRODUKT MIT DIESEM ÜBERTUBUS
OVERTUBE AND MEDICAL DEVICE USING THIS OVERTUBE

(30) Priorité: 22.07.2019 FR 1908305
(43) Date de publication de la demande: 01.06.2022
(73) Titulaire: Bastid, Christophe, 13009 Marseille (FR); Manos, Thierry, 13007 Marseille (FR)
(72) Inventeur: Bastid, Christophe, 13009 Marseille (FR); Manos, Thierry, 13007 Marseille (FR)
(74) Mandataire: Roman, Alexis
(86) Numéro de dépôt international: PCT/FR2020/051409
(87) Numéro de publication internationale: WO 2021/014110

(56) Documents cités:
- EP-A1- 3 409 316
- WO-A1-2018/203386
- JP-A- 2007 167 302
- US-A1- 2010 261 962
- US-B2- 8 343 034

## Description

### Domaine technique.

L'invention a pour objet un un surtube ainsi qu'un dispositif médical utilisant ce surtube.

L'invention se rapporte au domaine technique des accessoires permettant l'introduction d'endoscopes dans une cavité corporelle.

### Etat de la technique.

La « sleeve-gastrectomy » est une technique de chirurgie bariatrique invasive visant à réduire la taille de l'estomac avec pour but la perte de poids. Partie intégrante de l'endoscopie bariatrique, l' « endo-sleeve » est une technique endoscopique permettant de réaliser des plis dans la paroi gastrique et de suturer ces plis afin de réduire ainsi le volume de l'estomac (plicature gastrique transorale ou gastroplastie transorale).

Comme la paroi gastrique est relativement épaisse et dure, la suture des plis s'avère être une tâche difficile et longue et nécessite un matériel spécifiquement dédié. Des dispositifs médicaux dédiés à ce type d'intervention sont par exemple décrit dans les documents brevets US2016/0235400, US2007/0197862 ou US2005/0251166. Ces dispositifs médicaux utilisent un seul endoscope, souvent avec deux canaux opérateurs, permettant le passage de deux instruments chirurgicaux : un instrument pour pincer la paroi gastrique et former le pli ; et un autre instrument pour suturer ce pli. D'autre dispositifs médicaux utilisant un surtube qui sont pertinents rélatif au sujet des revendications 1 et 4 sont décrits dans les documents US 8 343 034 B2 et JP 2007 167302 A.

Ces dispositifs sont toutefois complexes à réaliser et relativement onéreux. Ils nécessitent donc un investissement financier important pour l'utilisateur.

Un objectif de l'invention est de remédier à cet état des choses. Un autre objectif de l'invention est de proposer un accessoire dont la conception est simple et peu onéreuse et permettant de réaliser très simplement une endoscopie bariatrique à moindre coûts.

### Présentation de l'invention.

La solution proposée par l'invention est un surtube comprenant :
- un corps tubulaire souple s'étendant longitudinalement et présentant une partie proximale et une extrémité distale ouverte,
- un premier conduit adapté pour loger un premier tube d'insertion d'un premier endoscope dans le corps, avec possibilité de déplacement longitudinal dudit premier tube dans ledit premier conduit et mouvement de rotation dudit premier tube autour de son axe longitudinal, lequel premier conduit est aménagé longitudinalement à l'intérieur dudit corps et débouche au niveau de l'extrémité distale dudit corps,
- un second conduit adapté pour loger un second tube d'insertion d'un second endoscope dans le corps, avec possibilité de déplacement longitudinal dudit second tube dans ledit second conduit et mouvement de rotation dudit second tube autour de son axe longitudinal, lequel second conduit est aménagé longitudinalement à l'intérieur dudit corps et débouche au niveau de l'extrémité distale dudit corps,
- un premier orifice aménagé au niveau de la partie proximale du corps pour l'introduction du premier tube d'insertion dans le premier conduit,
- un second orifice aménagé au niveau de la partie proximale du corps pour l'introduction du second tube d'insertion dans le second conduit,
- un troisième orifice aménagé au niveau de la partie proximale du corps pour l'introduction d'un lubrifiant dans le premier conduit et dans le second conduit,
- et dans lequel le premier conduit et le second conduit communiquent entre eux, sur toute leur longueur, par un passage de communication, lequel passage débouche au niveau du troisième orifice.

Le surtube (ou overtube en anglais) est un accessoire généralement utilisé pour insérer un seul tube d'insertion d'endoscope dans une cavité corporelle. Le surtube est notamment inséré dans l'œsophage, par la bouche du patient, jusqu'à l'estomac. Le surtube conforme à l'invention est maintenant configuré pour recevoir deux endoscopes. L'utilisateur peut donc utiliser ce nouveau surtube avec deux endoscopes classiques, comportant chacun une instrumentation spécifique et un dispositif de vision optique. L'utilisateur peut ainsi introduire dans la cavité corporelle (notamment dans l'estomac), deux instruments chirurgicaux distincts (un par endoscope) pour réaliser l'acte de chirurgie, chaque instrument pouvant être observé selon son propre axe de visionnage. L'utilisateur n'a plus besoin d'acheter un endoscope onéreux à double canal opérateur tels que décrit dans l'art antérieur précité. L'introduction du lubrifiant dans les des deux conduits se fait simplement depuis le troisième orifice, le passage d'ouverture entre les deux dits conduits permettant de les lubrifier simultanément. La conception du surtube est particulièrement simple, ce qui le rend peu onéreux et simple d'utilisation.

D'autres caractéristiques avantageuses de l'invention sont listées ci-dessous. Chacune de ces caractéristiques peut être considérée seule ou en combinaison avec les caractéristiques remarquables définies ci-dessus. Chacune de ces caractéristiques contribue, le cas échéant, à la résolution de problèmes techniques spécifiques définis plus avant dans la description et auxquels ne participent pas nécessairement les caractéristiques remarquables définies ci-dessus. Ces dernières peuvent faire l'objet, le cas échéant, d'une ou plusieurs demandes de brevet divisionnaires :
- le premier conduit, le second conduit et le passage de communication sont avantageusement configurés de sorte que le premier tube d'insertion du premier endoscope et le second tube d'insertion du second endoscope ne se touchent pas lorsque lesdits tubes sont logés dans lesdits conduits.
- Avantageusement, le passage de communication reliant le premier conduit au second conduit est plus étroit que lesdits conduits.
- Avantageusement, le corps présente une section transversale en forme de 8.
- Avantageusement, le corps a une longueur comprise entre 250 mm et 1500 mm.

Un autre aspect de l'invention concerne un dispositif médical comprenant :
- un premier endoscope comprenant :
   - - un premier tube d'insertion souple présentant une première partie distale équipée d'un béquillage, et à l'intérieur duquel est aménagé un premier canal opératoire pour l'insertion d'un premier instrument chirurgical (avantageusement une pince à mors larges), lequel premier canal débouche à une première extrémité distale dudit premier tube,
   - - un premier dispositif de vision optique situé au niveau de la première extrémité distale du premier tube d'insertion,
   - - une premier poignée de commande connectée à une extrémité proximale du premier tube d'insertion,
- un second endoscope comprenant :
   - - un second tube d'insertion souple présentant une seconde partie distale équipée d'un béquillage, et à l'intérieur duquel est aménagé un second canal opératoire pour l'insertion d'un second instrument chirurgical (avantageusement une agrafeuse chirurgicale, ou un clip monté), lequel second canal débouche à une seconde extrémité distale dudit second tube,
   - - un second dispositif de vision optique situé au niveau de la seconde extrémité distale du second tube d'insertion,
   - - une seconde poignée de commande connectée à une extrémité proximale du second tube d'insertion,
- un surtube conforme à l'une des caractéristiques précédentes et dans lequel :
   - - le premier tube d'insertion est logé dans le premier conduit, la première partie distale et la première extrémité distale dudit premier tube ressortant en dehors dudit premier conduit et du corps par l'extrémité distale dudit corps,
   - - le second tube d'insertion est logé dans le second conduit, la seconde partie distale et la seconde extrémité distale dudit second tube ressortant en dehors dudit second conduit et du corps par l'extrémité distale dudit corps.

### Brève description des figures.

D'autres avantages et caractéristiques de l'invention apparaîtront mieux à la lecture de la description d'un mode de réalisation préféré qui va suivre, en référence aux dessins annexés, réalisés à titre d'exemples indicatifs et non limitatifs et sur lesquels :
[Fig. 1] est une vue en perspective d'un endoscope traditionnel, utilisé dans l'invention, ainsi qu'un agrandissement de la tête dudit endoscope.
[Fig. 2] est un schéma d'un surtube selon l'invention.
[Fig. 3] est une vue en coupe selon A-A du surtube de la figure 2.
[Fig. 4] est une vue en perspective montrant des détails de conception d'un dispositif médical selon l'invention.
[Fig. 5] schématise la mise en place d'un dispositif médical selon l'invention, dans le corps d'un patient.
[Fig. 6] schématise la formation d'un pli dans la paroi gastrique d'un patient et sa suture, au moyen du dispositif médical selon l'invention.

### Description des modes de réalisation.

Par souci de clarté, les précisions suivantes sont apportées à certains termes utilisés dans la description et les revendications :
- « A et/ou B » signifie : A seul ou B seul ou A+B.
- sauf indication contraire, l'utilisation des adjectifs ordinaux «*premien*», *«second»,* etc., pour décrire un objet indique simplement que différentes occurrences d'objets similaires sont mentionnées et n'implique pas que les objets ainsi décrits doivent être dans une séquence donnée, que ce soit dans le temps, dans l'espace, dans un classement ou de toute autre manière.

Le dispositif médical selon l'invention comporte un surtube et deux endoscopes classiques. Il est notamment adapté pour réaliser une opération de chirurgie bariatrique, et plus spécifiquement une endoscopie bariatrique, mais peut être utilisé pour d'autres actes de chirurgie endoscopique.

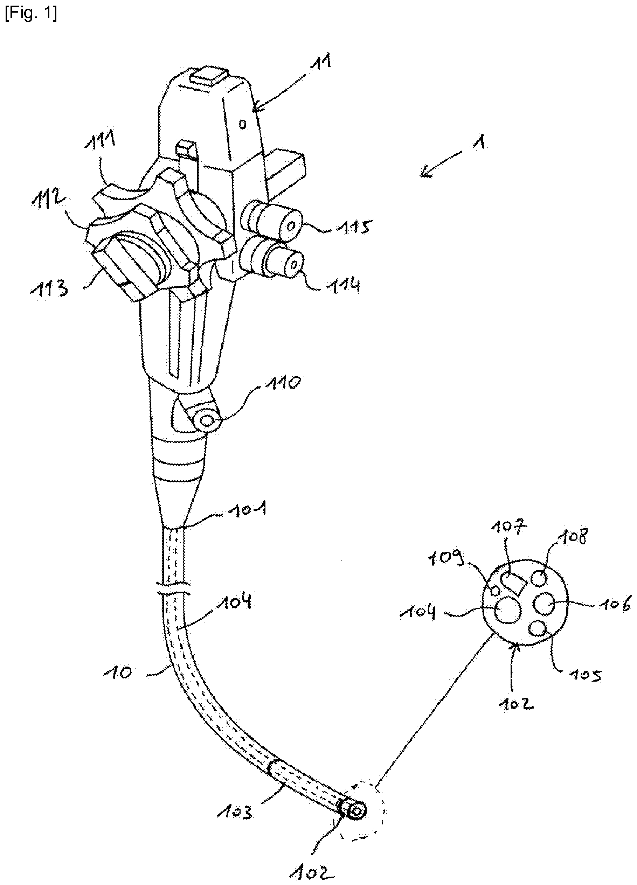

Un tel endoscope classique 1 est illustré sur la figure 1. Il s'agit d'un endoscope flexible, également désigné par sonde endoscopique ou fibroscope. Il permet d'explorer des cavités internes du corps du patient telles que bronches, oesophage, estomac, ou autre et d'intervenir chirurgicale sur une zone cible de celle.ci. Il comporte un tube d'insertion 10 et une poignée de commande 11.

Le tube d'insertion 10 est un tube souple s'étendant selon un axe longitudinal et qui est destiné à être inséré dans le corps du patient. Il est de forme généralement cylindrique et formé par une gaine réalisée dans un matériau plastique dont le diamètre externe est compris entre 3 mm et 13 mm. Le tube 10 présente une extrémité proximale 101 solidaire de la poigné de commande 11 et une extrémité distale 102 - ou tête - qui en usage est situé dans la cavité interne du corps du patient. La longueur du tube 10 entre les deux extrémités 101 et 102 est comprise entre 300 mm et 1500 mm. La tête 102 est disposée à l'extrémité d'une partie distale 103 équipée d'un béquillage commandé depuis la poignée de commande 11.

A l'intérieur du tube 10 est aménagé un canal opératoire 104. Il s'agit d'un canal qui s'étend longitudinalement dans le tube 10 et qui débouche au niveau de la tête 102. Ce canal 104 permet d'insérer un instrument chirurgical (pince, agrafeuse, ciseaux, brosse...) dans le tube 10 et de faire ressortir sa partie active (ou outil) au niveau de la tête 102. L'insertion de l'instrument chirurgical est effectué depuis un orifice d'entrée 110 situé au niveau de la poignée de commande 11 et qui débouche dans le canal 104. Sur la figure 1, ce canal opératoire 104 est unique. En d'autres termes, le tube 10 ne présente qu'un seul canal opératoire 104. Selon un mode de réalisation, le canal opératoire 104 peut être double, le tube 10 présentant alors deux canaux opératoires.

A l'intérieur du tube 10 est également aménagé un passage pour un dispositif de vision optique. Ce dispositif permet de former une image de la zone d'intervention dans la cavité. Il comporte une source de lumière 105 disposée sur la tête 102, par exemple formée d'une Led ou de fibres optiques, et pour illuminer la zone d'intervention. Il comporte également un capteur d'images 106 (ex : caméra CCD) disposé sur la tête 102 pour obtenir une image de la zone d'intervention éclairée.

Le tube 10 peut encore comporter, le cas échéant : un passage pour une injection d'eau débouchant au niveau de la tête 102 par un orifice d'injection 107 ; un passage pour une insufflation d'air débouchant au niveau de la tête 102, par un orifice d'insufflation 108 ; un passage pour une aspiration de fluide débouchant au niveau de la tête 102, par un orifice d'aspiration 109.

La poignée de commande 11 est adaptée pour être connecté à un connecteur (lumière, vidéo) par l'intermédiaire d'un cordon de liaison (non représenté). Sur la figure 1, elle comporte des manettes de commande du béquillage de la partie distale 103. Une manette 111 pour un déplacement haut/bas du béquillage et une manette 112 pour un déplacement droite/gauche dudit béquillage. Les câbles reliant les manettes 111, 112 au béquillage passent au travers du tube 10. Lorsque l'utilisateur manoeuvre ces manettes, il peut actionner la déformation et/ou l'angulation de la partie distale 103 et partant de la tête 102. Il peut ainsi modifier l'orientation dans l'espace de cette dernière. Les manettes 111, 112 peuvent coopérer avec un frein 113 permettant de bloquer en position le béquillage. Sur la figure 1, la poignée de commande 11 est également pourvu d'un connecteur 114 pour l'injection eau/air et d'un connecteur 115 pour l'aspiration des fluides.

Les deux endoscopes du dispositif médical sont similaires à celui décrit en référence à la figure 1. Dans la suite de la description, le premier endoscope est associé à la référence « A » et le second endoscope à la référence « B ».

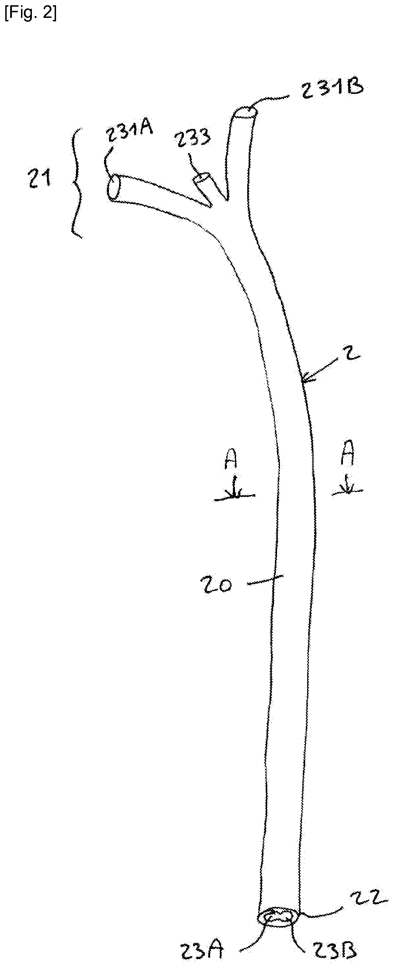

Un surtube 2 conforme à l'invention est illustré sur les figures 2 à 4. Il comporte un corps tubulaire 20 qui est souple et qui s'étend longitudinalement. Le corps 20 est par exemple réalisé en silicone ou polyuréthane et obtenu par extrusion ou moulage. Il présente une partie proximale 21 et une extrémité distale ouverte 22. A titre d'exemple, la longueur du corps 20 entre les deux extrémités 21 et 22 est par exemple comprise entre 250 mm et 1500 mm ; son diamètre externe, ou dimension externe, est compris entre 10 mm et 30 mm ; et son épaisseur comprise entre 1 mm et 3 mm.

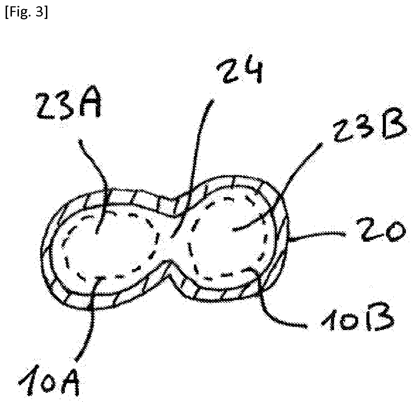

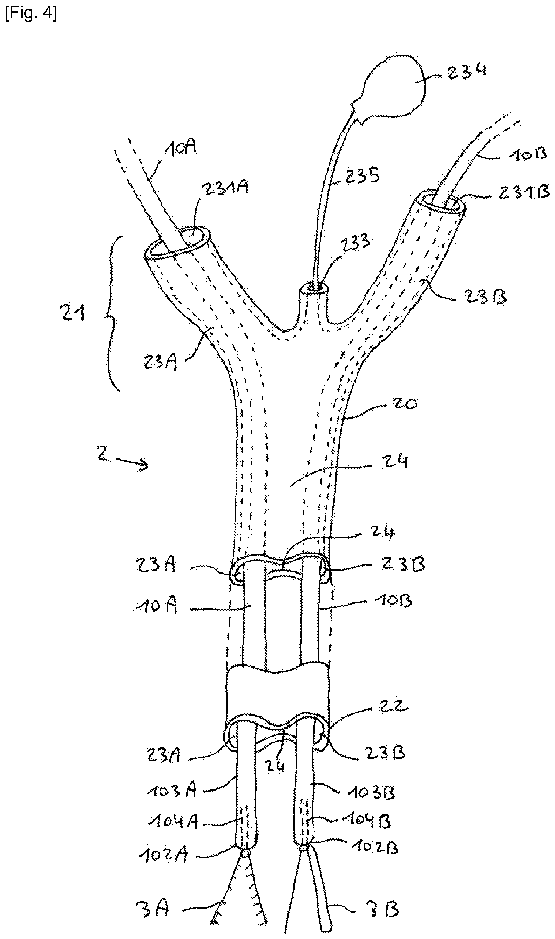

En se rapportant aux figures 3 et 4, deux conduits 23A, 23B sont aménagés longitudinalement dans le corps 20. Le premier conduit 23A est adapté pour loger le premier tube d'insertion 10A du premier endoscope 1A. Et le second conduit 23B est adapté pour loger le second tube d'insertion 10B du second endoscope 1B. Ces deux conduits 23A, 23B débouchent au niveau de l'extrémité distale 22.

Les conduits 23A, 23B sont configurées de manière à ce que les tubes 10A, 10B puissent se déplacer longitudinalement dans le corps 20. En d'autres termes, les tubes 10A, 10B ont un mouvement de translation haut/bas dans le corps 20. Les dimensions internes (diamètres) des conduits 23A, 23B correspondent sensiblement aux dimensions externes (diamètres) des tubes 10A, 10B, en étant toutefois légèrement supérieures, par exemple de 0,5 à 1 mm, pour faciliter le déplacement desdits tubes selon l'axe longitudinal du corps 20. Les conduits 23A, 23B autorisent également un mouvement de rotation des tubes 10A, 10B de sorte que lesdits tubes peuvent pivoter sur eux-mêmes autour de leur axe longitudinal, à l'intérieur de leur conduit respectif 23A, 23B.

Sur les figures 2 et 4, la partie proximale 21 du corps 20 a une forme générale en W. Elle comporte un premier orifice 231A pour l'introduction du premier tube 10A dans le premier conduit 23A et un second orifice 231B pour l'introduction du second tube 10B dans le second conduit 23B. Un troisième orifice 233 est également aménagé au niveau de la partie proximale 21. Ce troisième orifice 233 sert à introduire un lubrifiant dans les deux conduits 23A, 23B. Ce lubrifiant est par exemple de l'huile de paraffine ou de la glycérine. Il permet de réduire les frottements entre la paroi interne des conduits 23A, 23B et la paroi externe des tubes 10A, 10B, de manière à faciliter l'insertion desdits tubes dans le corps 20 et leur déplacement longitudinal. Le troisième orifice 233 a par exemple un diamètre compris entre 1 mm et 10 mm. Le lubrifiant utilisé peut être contenu dans un réservoir souple 234 de type poire ou seringue, relié au troisième orifice 233 par un conduit 235. En exerçant manuellement une pression sur le réservoir 234 ou la seringue, le lubrifiant est dispensé dans le corps 20.

Sur les figures 3 et 4, le premier conduit 23A et le second conduit 23B communiquent entre eux, au moins dans la longueur du corps 20 qui n'inclue pas la partie proximale 21, par un passage de communication 24. Sur les figures 3 et 4, les conduits 23A, 23B sont séparés et ne sont plus adjacents dans la partie proximale 21. Selon un mode de réalisation, les conduits 23A, 23B sont adjacents sur toute leur longueur, y compris dans la partie proximale 21, et communiquent entre eux, sur toute leur longueur, par le passage 24. Ce passage 24 débouche au niveau du troisième orifice 233. Ainsi, lorsque le lubrifiant est introduit dans le corps 20 depuis le troisième orifice 233, il va se rependre par gravité simultanément dans les deux conduits 23A, 23B, sur toute leur longueur, de sorte que lesdits conduits sont parfaitement lubrifiés.

Le premier conduit 23A, le second conduit 23B et le passage 24 sont configurés de sorte que le corps 20 présente une section transversale en forme de 8 (figure 3). Cette configuration évite toute surépaisseur de la paroi du corps 20, ce qui permet non seulement d'optimiser son encombrement, mais également d'en réduire le coût de fabrication.

Le premier conduit 23A, le second conduit 23B et le passage 24 sont également configurés de sorte que les deux tubes 10A et 10B ne se touchent pas lorsqu'ils sont logés dans lesdits conduits. On évite ainsi tout chevauchement et frottement des tubes 10A, 10B entre eux, susceptibles de gêner leur insertion et/ou déplacement dans le corps 20. Préférentiellement, pour obtenir cet effet technique tout en simplifier la conception du corps 20, le passage 24 est plus étroit que les conduits 23A, 23B.

Sur la figure 4, lorsque les tubes d'insertion 10A, 10B sont installés dans le corps 20 et logés dans leur conduit respectifs 23A, 23B, leur partie distale 103A, 103B et leur tête 102A, 102B ressortent en dehors desdits conduits et dudit corps, par l'extrémité distale 22 de ce dernier. Les parties distales 103A, 103B et les têtes 102A, 102B font ainsi saillie du corps 20 pour, en usage, être positionnées dans la cavité corporelle.

Sur la figure 4, l'instrument 3A inséré dans le canal opératoire 104A du premier tube 10A du premier endoscope 1A est une pince, préférentiellement une pince à mors larges (largeur comprise entre 3 mm et 10 mm), qui est non traumatisante. L'instrument 3B inséré dans le canal opératoire 104B du second tube 10B du second endoscope 1B est une agrafeuse chirurgicale ou un clip monté, assurant la pose simultanée d'une ou plusieurs agrafes ou clips (par exemple la pose simultanée de plusieurs rangées d'agrafes ou clips). Les agrafes ou clips utilisés sont préférentiellement réalisés en titane. A titre d'exemple indicatif, leur largeur est d'environ 3 mm et leur longueur comprise entre 3 mm à 5 mm. Ce type d'agrafeuse chirurgicale est par exemple commercialisé par les sociétés Ethicon Endo Surgery^{®} ou Covidien^{®}. En usage, les instruments 3A, 3B sortent de leur canal opératoire respectif 104a, 104B, et sont situés en dehors du corps 20 pour être positionnés dans la cavité corporelle.

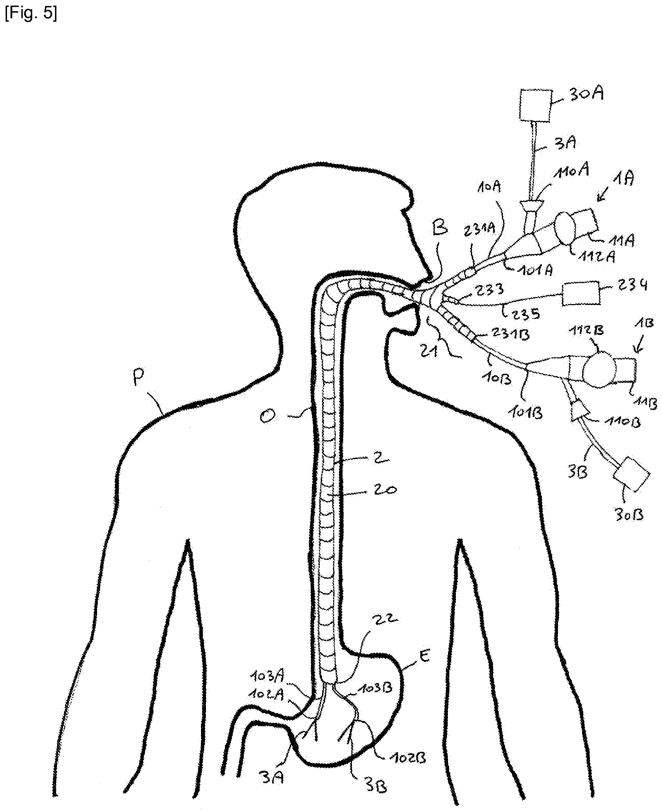

La figure 5 illustre la mise en place du dispositif médical dans le corps d'un patient P pour une opération de chirurgie bariatrique. Le surtube 2 est d'abord inséré depuis la bouche B du patient P, dans l'œsophage O, jusqu'à ce que l'extrémité distale 22 du corps 20 soit située dans l'estomac E. Cette insertion peut être réalisée sur un fil-guide mis en place initialement sous contrôle d'une gastroscopie. Lorsque le surtube 2 est installé, sa partie proximale 21 est située en dehors de la bouche B du patient P, de sorte que les orifices 231A, 231B et 233 sont accessibles.

On insère alors dans le corps 20, le premier tube 10A du premier endoscope 1A. Cette insertion est réalisée depuis l'orifice d'entrée 231A de sorte que le premier tube 10 soit logé dans le premier conduit 23A. Le tube 10A et/ou le premier conduit 23A sont avantageusement préalablement lubrifiés. Cette insertion est finalisée lorsque la partie distale 103A et la tête 102A du premier tube 10A ressortent du premier conduit 23A et sont situées dans l'estomac E, à proximité de la zone d'intervention. Cette insertion peut être réalisée sous contrôle du dispositif de vision optique du premier endoscope 1A. On insère de la même façon le second tube 10B du second endoscope 1B.

Le premier instrument 3A est ensuite inséré dans le canal opératoire 104A du premier tube 10A par l'orifice d'entrée 110A. Cette insertion est finalisée lorsque l'outil (ex : pince) du premier instrument 3A ressort de la tête 102A pour être situés dans l'estomac E. On insère de la même façon le second instrument 3B dans le canal opératoire 104B du second tube 10B du second endoscope 1B. Les instruments 3A, 3B sont actionnables depuis des organes de commande 30A, 30B situés en dehors du corps du patient P.

Si l'utilisateur a besoin d'approcher ou d'éloigner de la zone d'intervention les outils des instruments 3A, 3B, il peut manipuler les endoscopes 1A, 1B pour faire coulisser et/ou pivoter les tubes 10A, 10B dans leur conduits respectifs 23A, 23B. Ces déplacements s'accompagnent préférentiellement d'une lubrification des conduits 23A, 23B depuis le troisième orifice 233.

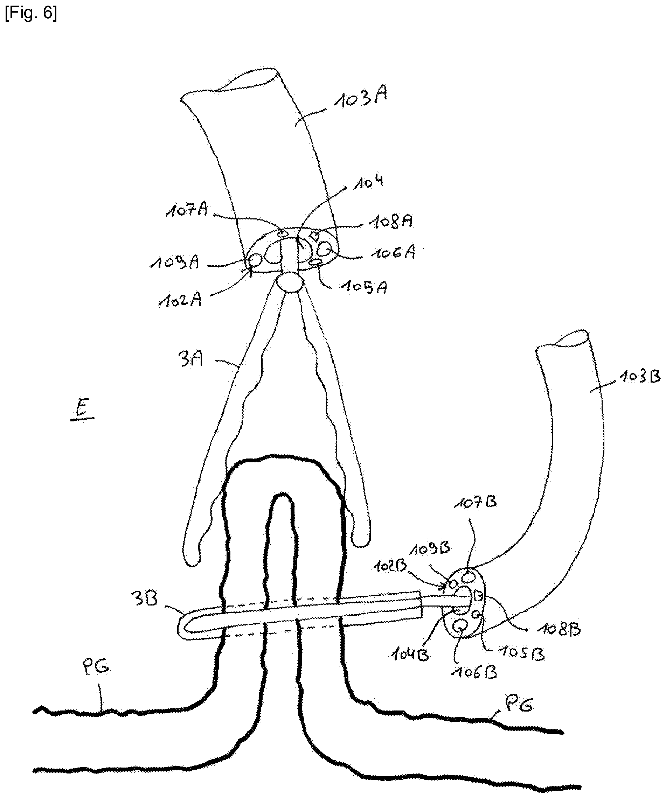

Sur la figure 6, les outils des instruments 3A et 3B (respectivement une pince et une agrafeuse chirurgicale) sont positionnés au niveau de la zone d'intervention. Cette zone est ici une région de la paroi gastrique PG (péritoine). Le béquillage des parties distales 103A, 103B permet d'orienter spécifiquement chaque instrument 3A, 3B, la pince et l'agrafeuse étant ici orienté à 90°. Chaque instrument 3A, 3B est en outre regardé par leur propre caméra 106A, 106B. L'utilisateur a donc une meilleure vision de la zone d'intervention qu'il peut observer sous deux angles différents.

Sur la figure 6, la pince 3A est positionnée perpendiculairement à la paroi PG. Cette pince est actionnée pour pincer la paroi PG et venir former un pli large qui peut emporter les feuillets péritonéaux. L'agrafeuse ou le clip monté 3B est positionné perpendiculairement à la pince 34 et de fait, perpendiculairement au pli ainsi formé. L'agrafeuse ou le clip monté 3B est alors actionné pour agrafer le pli et le maintenir en position. Il n'y a aucune couture de la paroi PG mais simplement un maintien par clips ou agrafes, ce qui est beaucoup plus rapide et moins traumatisant pour ladite paroi, sans risque d'effraction péritonéale.

Une ou plusieurs caractéristiques exposées seulement dans un mode de réalisation peuvent être combinées avec une ou plusieurs autres caractéristiques exposées seulement dans un autre mode de réalisation.

L'agencement des différents éléments et/ou moyens et/ou étapes de l'invention, dans les modes de réalisation décrits ci-dessus, ne doit pas être compris comme exigeant un tel agencement dans toutes les implémentations. Diverses variantes peuvent être prévues, et notamment :
- La pièce 33 n'a pas nécessairement la forme d'un tube, mais peut être de forme sphérique, ovoïdale, etc.
- La pièce 33 n'est pas nécessairement en matériau plastique mais peut également être en résine, en métal, en composite, en matériau végétal, etc.

## Revendications

1. Surtube comprenant :
- un corps tubulaire souple (20) s'étendant longitudinalement et présentant une partie proximale (21) et une extrémité distale (22) ouverte,
- un premier conduit (23A) adapté pour loger un premier tube d'insertion (10A) d'un premier endoscope (1A) dans le corps (20), avec possibilité de déplacement longitudinal dudit premier tube dans ledit premier conduit et mouvement de rotation dudit premier tube autour de son axe longitudinal, lequel premier conduit (23A) est aménagé longitudinalement à l'intérieur dudit corps et débouche au niveau de l'extrémité distale (22) dudit corps,
- un second conduit (23B) adapté pour loger un second tube d'insertion (10B) d'un second endoscope (1B) dans le corps (20), avec possibilité de déplacement longitudinal dudit second tube dans ledit second conduit et mouvement de rotation dudit second tube autour de son axe longitudinal, lequel second conduit (23B) est aménagé longitudinalement à l'intérieur dudit corps et débouche au niveau de l'extrémité distale (22) dudit corps,
- un premier orifice (231A) aménagé au niveau de la partie proximale (21) du corps (20) pour l'introduction du premier tube d'insertion (10A) dans le premier conduit (23A),
- un second orifice (231B) aménagé au niveau de la partie proximale (21) du corps (20) pour l'introduction du second tube d'insertion (10B) dans le second conduit (23B),
- un troisième orifice (233) aménagé au niveau de la partie proximale (21) du corps pour l'introduction d'un lubrifiant dans le premier conduit (23A) et dans le second conduit (23B),
et dans lequel le premier conduit (23A) et le second conduit (23B) communiquent entre eux, au moins dans la longueur du corps (20) qui n'inclue pas la partie proximale (21), par un passage de communication (24), lequel passage débouche au niveau du troisième orifice (233), dans lequel le corps (20) présente une section transversale en forme de 8.

2. Surtube selon la revendication 1, le premier conduit (23A), le second conduit (23B) et le passage de communication (24) sont configurés de sorte que le premier tube d'insertion (10A) du premier endoscope (1A) et le second tube d'insertion (10B) du second endoscope (1B) ne se touchent pas lorsque lesdits tubes sont logés dans lesdits conduits.

3. Surtube selon l'une des revendications précédentes, dans lequel le corps (20) a une longueur comprise entre 250 mm et 1500 mm.

4. Dispositif médical comprenant :
- un premier endoscope (1A) comprenant :
- un premier tube d'insertion (10A) souple présentant une première partie distale (103A) équipée d'un béquillage, et à l'intérieur duquel est aménagé un premier canal opératoire (104A) pour l'insertion d'un premier instrument chirurgical (3A), lequel premier canal débouche à une première extrémité distale (102A) dudit premier tube,
- un premier dispositif de vision optique (105A, 106A) situé au niveau de la première extrémité distale (102A) du premier tube d'insertion (10A),
- une premier poignée de commande (11A) connectée à une extrémité proximale (110A) du premier tube d'insertion (10A),
- un second endoscope (1B) comprenant :
- un second tube d'insertion (10B) souple présentant une seconde partie distale (103B) équipée d'un béquillage, et à l'intérieur duquel est aménagé un second canal opératoire (104B) pour l'insertion d'un second instrument chirurgical (3B), lequel second canal débouche à une seconde extrémité distale (102B) dudit second tube,
- un second dispositif de vision optique (105B, 106B) situé au niveau de la seconde extrémité distale (102B) du second tube d'insertion (10B),
- une seconde poignée de commande (11B) connectée à une extrémité proximale (110B) du second tube d'insertion (10B),
- un surtube (2) conforme à la revendication 1 et dans lequel :
- le premier tube d'insertion (10A) est logé dans le premier conduit (23A), la première partie distale (103A) et la première extrémité distale (102A) dudit premier tube ressortant en dehors dudit premier conduit et du corps (20) par l'extrémité distale (22) dudit corps,
- le second tube d'insertion (10B) est logé dans le second conduit (23B), la seconde partie distale (103B) et la seconde extrémité distale (102B) dudit second tube ressortant en dehors dudit second conduit et du corps (20) par l'extrémité distale (22) dudit corps,
dans lequel le corps (20) présente une section transversale en forme de 8.

5. Dispositif médical selon la revendication 4, dans lequel le premier instrument chirurgical (3A) est une pince à mors larges.

6. Dispositif médical selon l'une des revendications 4 ou 5, dans lequel le second instrument chirurgical (3B) est une agrafeuse chirurgicale, ou un clip monté.

## Patentansprüche

1. Übertubus, welcher umfasst:
- einen flexiblen rohrförmigen Körper (20), der sich in Längsrichtung erstreckt und einen proximalen Teil (21) und ein offenes distales Ende (22) aufweist,
- einen ersten Kanal (23A), der dazu eingerichtet ist, einen ersten Einführschlauch (10A) eines ersten Endoskops (1A) im Körper (20) aufzunehmen, mit der Möglichkeit einer Längsverschiebung des ersten Schlauches im ersten Kanal und einer Drehbewegung des ersten Schlauches um seine Längsachse, wobei dieser erste Kanal (23A) im Inneren des Körpers in Längsrichtung angeordnet ist und am distalen Ende (22) des Körpers mündet,
- einen zweiten Kanal (23B), der dazu eingerichtet ist, einen zweiten Einführschlauch (10B) eines zweiten Endoskops (1B) im Körper (20) aufzunehmen, mit der Möglichkeit einer Längsverschiebung des zweiten Schlauches im zweiten Kanal und einer Drehbewegung des zweiten Schlauches um seine Längsachse, wobei dieser zweite Kanal (23B) im Inneren des Körpers in Längsrichtung angeordnet ist und am distalen Ende (22) des Körpers mündet,
- eine erste Öffnung (231A), die am proximalen Teil (21) des Körpers (20) angeordnet ist, zur Einführung des ersten Einführschlauches (10A) in den ersten Kanal (23A),
- eine zweite Öffnung (231B), die am proximalen Teil (21) des Körpers (20) angeordnet ist, zur Einführung des zweiten Einführschlauches (10B) in den zweiten Kanal (23B),
- eine dritte Öffnung (233), die am proximalen Teil (21) des Körpers angeordnet ist, zur Einführung eines Schmiermittels in den ersten Kanal (23A) und in den zweiten Kanal (23B),
und wobei der erste Kanal (23A) und der zweite Kanal (23B) wenigstens in der Länge des Körpers (20), welche den proximalen Teil (21) nicht eischließt, über einen Verbindungsdurchgang (24) miteinander in Verbindung stehen, wobei dieser Durchgang an der dritten Öffnung (233) mündet, wobei der Körper (20) einen Querschnitt mit der Form einer "8" aufweist.

2. Übertubus nach Anspruch 1, wobei der erste Kanal (23A), der zweite Kanal (23B) und der Verbindungsdurchgang (24) so ausgebildet sind, dass sich der erste Einführschlauch (10A) des ersten Endoskops (1A) und der zweite Einführschlauch (10B) des zweiten Endoskops (1B) nicht berühren, wenn die Schläuche in den Kanälen aufgenommen sind.

3. Übertubus nach einem der vorhergehenden Ansprüche, wobei der Körper (20) eine Länge zwischen 250 mm und 1500 mm aufweist.

4. Medizinprodukt, welches umfasst:
- ein erstes Endoskop (1A), welches umfasst:
- einen ersten flexiblen Einführschlauch (10A), der einen mit einer Abwinkelung ausgestatteten ersten distalen Teil (103A) aufweist und in dessen Innerem ein erster Operationskanal (104A) zur Einführung eines ersten chirurgischen Instruments (3A) angeordnet ist, wobei dieser erste Kanal an einem ersten distalen Ende (102A) des ersten Schlauches mündet,
- eine erste optische Sichtvorrichtung (105A, 106A), die sich am ersten distalen Ende (102A) des ersten Einführschlauches (10A) befindet,
- einen ersten Steuergriff (11A), der mit einem proximalen Ende (110A) des ersten Einführschlauches (10A) verbunden ist,
- ein zweites Endoskop (1B), welches umfasst:
- einen zweiten flexiblen Einführschlauch (10B), der einen mit einer Abwinkelung ausgestatteten zweiten distalen Teil (103B) aufweist und in dessen Innerem ein zweiter Operationskanal (104B) zur Einführung eines zweiten chirurgischen Instruments (3B) angeordnet ist, wobei dieser zweite Kanal an einem zweiten distalen Ende (102B) des zweiten Schlauches mündet,
- eine zweite optische Sichtvorrichtung (105B, 106B), die sich am zweiten distalen Ende (102B) des zweiten Einführschlauches (10B) befindet,
- einen zweiten Steuergriff (11B), der mit einem proximalen Ende (110B) des zweiten Einführschlauches (10B) verbunden ist,
- einen Übertubus (2) gemäß Anspruch 1, wobei:
- der erste Einführschlauch (10A) in dem ersten Kanal (23A) aufgenommen ist, wobei der erste distale Teil (103A) und das erste distale Ende (102A) des ersten Schlauches am distalen Ende (22) des Körpers aus dem ersten Kanal und dem Körper (20) herausragen,
- der zweite Einführschlauch (10B) in dem zweiten Kanal (23B) aufgenommen ist, wobei der zweite distale Teil (103B) und das zweite distale Ende (102B) des zweiten Schlauches am distalen Ende (22) des Körpers aus dem zweiten Kanal und dem Körper (20) herausragen,
wobei der Körper (20) einen Querschnitt mit der Form einer "8" aufweist.

5. Medizinprodukt nach Anspruch 4, wobei das erste chirurgische Instrument (3A) eine Zange mit breiten Backen ist.

6. Medizinprodukt nach einem der Ansprüche 4 oder 5, wobei das zweite chirurgische Instrument (3B) eine chirurgische Klammervorrichtung oder ein vormontierter Clip ist.

## Claims

1. Overtube comprising:
- a flexible tubular body (20) extending longitudinally and having a proximal part (21) and an open distal end (22),
- a first duct (23A) designed to house a first tube (10A) for insertion of a first endoscope (1A) into the body (20), with the possibility of longitudinal displacement of said first tube in said first duct and rotational movement of said first tube about its longitudinal axis, which first duct (23A) is formed longitudinally inside said body and opens at the distal end (22) of said body,
- a second duct (23B) designed to house a second tube (10B) for insertion of a second endoscope (1B) into the body (20), with the possibility of longitudinal displacement of said second tube in said second duct and rotational movement of said second tube about its longitudinal axis, which second duct (23B) is formed longitudinally inside said body and opens at the distal end (22) of said body,
- a first orifice (231A) formed at the proximal part (21) of the body (20) for the introduction of the first insertion tube (10A) into the first duct (23A),
- a second orifice (231B) formed at the proximal part (21) of the body (20) for the introduction of the second insertion tube (10B) into the second duct (23B),
- a third orifice (233) formed at the proximal part (21) of the body for the introduction of a lubricant into the first duct (23A) and into the second duct (23B),
and wherein the first duct (23A) and the second duct (23B) communicate with each other, at least along the length of the body (20) that does not include the proximal part (21), via a communication passage (24), which passage opens at the third orifice (233), wherein the body (20) has a cross section in the shape of an 8.

2. Overtube according to Claim 1, the first duct (23A), the second duct (23B) and the communication passage (24) are configured such that the first insertion tube (10A) of the first endoscope (1A) and the second insertion tube (10B) of the second endoscope (1B) do not touch when said tubes are housed in said ducts.

3. Overtube according to either of the preceding claims, wherein the body (20) has a length of between 250 mm and 1500 mm.

4. Medical device comprising:
- a first endoscope (1A) comprising:
- a first flexible insertion tube (10A) having a first distal part (103A) equipped with tip deflection, and inside which is formed a first operating channel (104A) for the insertion of a first surgical instrument (3A), which first channel opens at a first distal end (102A) of said first tube,
- a first optical vision device (105A, 106A) situated at the first distal end (102A) of the first insertion tube (10A),
- a first control handle (11A) connected to a proximal end (110A) of the first insertion tube (10A),
- a second endoscope (1B) comprising:
- a second flexible insertion tube (10B) having a second distal part (103B) equipped with tip deflection, and inside which is formed a second operating channel (104B) for the insertion of a second surgical instrument (3B), which second channel opens at a second distal end (102B) of said second tube,
- a second optical vision device (105B, 106B) situated at the second distal end (102B) of the second insertion tube (10B),
- a second control handle (11B) connected to a proximal end (110B) of the second insertion tube (10B),
- an overtube (2) in accordance with Claim 1 and in which:
- the first insertion tube (10A) is housed in the first duct (23A), the first distal part (103A) and the first distal end (102A) of said first tube emerging outside said first duct and the body (20) via the distal end (22) of said body,
- the second insertion tube (10B) is housed in the second duct (23B), the second distal part (103B) and the second distal end (102B) of said second tube emerging outside said second duct and the body (20) via the distal end (22) of said body,
wherein the body (20) has a cross section in the shape of an 8.

5. Medical device according to Claim 4, wherein the first surgical instrument (3A) is a clamp with wide jaws.

6. Medical device according to either of Claims 4 and 5, wherein the second surgical instrument (3B) is a surgical stapler or a mounted clip.
